# EUROPEAN PATENT SPECIFICATION

(11) **EP 1 028 675 B1**
(45) Date of publication and mention of the grant of the patent: **11.02.2004**
(21) Application number: 98920801.2
(22) Date of filing: 29.04.1998
(51) Int. Cl.: A61F 2/68

(54) **ARTIFICIAL SENSIBILITY**
KÜNSTLICHE EMPFINDLICHKEIT
SENSIBILITE ARTIFICIELLE

(30) Priority: 29.04.1997 SE 9701595
(43) Date of publication of application: 23.08.2000
(73) Proprietor: Handevelop AB, 240 13 Genarp (SE)
(72) Inventor: LUNDBORG, Göran, S-240 13 Genarp (SE)
(74) Representative: Perneborg, Henry T.
(86) International application number: PCT/SE1998/000786
(87) International publication number: WO 1998/048740

(56) References cited:
- ISA BM 74303 (9-10) 1974, JACK B. JOHNSON et al., "A Tactile Prosthetic Device for the Denervated Hand".

## Description

### Field of the ivention

The present invention relates to a new principle for artificial sensibility by sense substitution (replacement of a lost sense function by an alternative sense). The primary goal is to, by use of the hearing sense, create sensibility in hand prostheses. The aim is also to substitute lost sensibility due to nerve injuries or any other type of lesion or illness, resulting in sensory loss or sensory impairment in the hand, parts of the hand, the foot or some other body-part. By the use of this principle the patient can "feel with the hearing sense" and can utilize the hearing sense to register and identify the surface, texture, density and shape of the item which is touched.

### Background of the invention

The human hand is a grip organ characterized by good grip power as well as capacity for fine motor precision movements. The hand is also a sense organ with an extremely well developed sensory function. Hand sensibility includes not only capacity to feel pressure, touch, vibration, temperature, changes etc but also capacity to - without vision - perceive the form, surface and texture of items that are touched a capacity named stereognosis. It is well known that hand function to a large extent is dependent on this sensory feedback system - "a hand without sensibility is a hand without function".

There are may situations where lack of sensibility constitutes a major hinder for function in the hand and where some kind of artificial sensibility would be of great importance. A review a number of such situations follows below:

Following amputation injuries at forearm - or upper arm level the patient can have good use of an "artificial hand", i.e. a hand prosthesis can replacing the amputated hand. Such a hand prosthesis can be fixed to the amputation stump by the use of a sleeve enclosing the remaining part of the forearm (amputation stump, or by titanium fixtures (according to Branemark) which are implanted into the skeleton. The prosthesis can be of cosmetic nature without possibilities for movement, or of functional type with possibilities for active volontary motion. The latter case refers to muscle-controlled myoelectric prostheses. These prostheses are controlled by electric activity in muscles which remain in the amputation stump. By surface electrodes in the sleeve, enclosing the amputation stump, such impulses in extensor or flexor muscles can be recorded and used for control of a motor in the prosthesis which can open or close the prosthetic hand.

Myoelectric hand prostheses can be very useful, but many patients use their prostheses only to a limited extent, or nor at all. The direct reason is often the fact that the prostheses lack sensibility so there is no feedback function in the system. Many patients are dependent on visual observation of every movement in the prosthesis in order to, to some extent, compensate for the lack of sensibility. A system which would give real sensibility in such a hand prosthesis would dramatically improve its function.

Severe transection or crush injuries in the arm or hand usually include lesions of one or several nerve trunks. For instance, transection of one of the major nerve trunks on the volar part of the wrist (median nerve) results in total sensory loss within the major part of the hand. Such an injury results in major disability since hand function is very much impaired due to the sensory loss. Following surgical repair of the nerve new nerve fibers grow into the hand, by because of misorientation of nerve fibers and incomplete innervation the sensibility often remains impaired in the hand. Following injury and repair of nerve trunks at wrist level about six months are required before there is any useful sensory recovery in the hand. During these months, when there is a complete sensory loss of the hand, the patient has great difficulties in exercising the hand since there is no sensory feedback. A system for articificial sensibility during this period would to a large extent facilitate the postoperative training which would result in more rapid rehabilitation.

A large number of neurological diseases result in impaired sensibility or total sensory loss in the hand. In polyneuropathies there may be such a sensory impairment in many parts of the body inlcuding the arms and hands. Such a neuropathy may exist in, e.g. diabetes, alcohol abuse or impaired kidney function. Impaired sensibility can be a problem also in MS (multiple sclerosis) and stroke. In industrial socities sensory impairment due to long-term use of handheld vibrating tools may be a major problem. In a global perspective leprosy is the most obvious example of how sensory impairment results not only in impaired hand function, but also in injuries and infections which may lead to spontaneous amputations of fingers, toes and other body-parts.

In these situations restitution of sensibility in the hand and other body-parts would represent a major advantage in terms of improved function, and would also help to prevent injuries to the body.

The discription, given above, has primarily addressed the hand and the upper extremity. Also in the *lower* extremity sensory loss may give major problems. In diabetes sensory loss in the foot may lead to wounds, infection, necrosis and amputation. In amputation injuries prostheses may be very useful, but because of lack of sensibility in the prostheses their use may be limited.

Artificial sensibility is a principle where lost or impaired sensibility is replaced by an alternative system for sensory feedback. To achieve such a goal the following is needed: 1) Detection of a sensory stimuli, e.g. pressure or vibration; 2) processing and transmission of the signal resulting from such a stimuli; 3) some type of sensory perception based on the transmitted signal.

Registration of a stimulus requires some type of sensor. For detection of *pressure* piezoresistive membranes have been used, e.g. in the grip organs of industrial robots. Also in experiments on humans piezoresistive membranes, applicated to the fingertips of hand prostheses or denervated hands have been used. A pressure, applied to such a membrane, initiate an electric signal which is proportional to the applied pressure.

The electric signals can be processed and used for application of electric or vibrotactile stimuli to intact, sensible skin more proximally in the same extremity or at some other part of the body. This principle, however, has major drawbacks, for instance due to rapid adaptation of the skin to such a stimulus. In addition, such a principle gives an "unphysiological" stimulus which not can be used for identification of the surface or structure that is touch.

### Object of the invention

The object of the present invention is to create possibilities to "feel with the hearing sense", i.e. to replace vibrotactile sensory stimuli with acoustic stimuli. The invention creates possibilities to, by using the hearing sense, identify the character of the surface and texture which is touched, and to hereby achieve a descriptive functional sensory perception. The invention is based on a new principle for artificial sensibility based on sense substitution i.e. the use of an alternative sense to replace a lost sense.

Various types of sense substitution exist in daily life: a blind person can read in Braille with the fingertips (vision is replaced by sensibility); a deaf person can read the movements of the lips of the contrahent (hearing sense is replaced by vision) and can also feel and utilize the vibrations in the larynx of the contrahent (hearing sense is replaced by the sensibility).

In the prevailing invention lost sensibility is replaced by the *hearing sense.* The hearing sense is extremely well developed and mimics in that respect the sensory sense. Sensory sense as well as hearing sense is to a great extent based on the detection and interpretation of vibration stimuli - substitution of lost sensibility by hearing is therefore a biologically sound principle.

Our aim is to use a principle which not only allows registration and perception of changes in threshold levels, temperature etc, but which also indeed makes possible registration and perception of the character of the surface and texture which is touched and which hereby can replace the descriptive, functional sensibility which characterizes the human hand.

### Summary of the invention

The main principle of the prevailing invention is to replace absent of lost sensibility in hand prostheses, hands or other body-part by the use of an alternate sense - the hearing sense - and to hereby use the special qualities of the hearing sense to replace lost sensibility. When a surface or an item normally is touched by a hand vibrations, represented by a weak friction sound, are always created. This friction sound is in each separate case very characteristic for the surface and texture which is touched. Normally this sound is very week. In case of lost or absent sensibility the prevailing invention can, however, utilize these sound stimuli, magnify them and process them to acoustic stimuli, the character of which is not falsified but rather represents an increased perception of the natural "sound of touch". We are not dealing with a falsified, unphysiological sound characterized by tones etc.

If the signal is processed in a stereo amplifier, and if both ears (alternatively bone conduction of the both sides of the cranium) are utilized, the fingers of the hand can be "projected" in different spatial localisations in space (like the sections of the symphony orchestra) which makes it possible for the patient to identify which fingers are touched. The principle can be combined with other systems for registration and perception of changes also with respect to touch, temperature etc.

The system requires a sensor system at the location for the touch, processing and amplifying components and an equipment for stimulation of the hearing sense or the bone conduction system of the cranium.

In the prevailing invention the sensors are microphones which can register "the friction sound" (vibrotactile stimulus) which is always released when an item is touched, and which is characteristic for its surface, texture and in some cases form and density. The "sound of touch" (which always can be heard faintly) is totally different when various materials are touched e.g. metal, glas, rubber foam, linen, an orange or an apple. In a hand prosthesis microphones can be incorporated directly in the fingers of the prosthesis. In regard to hands with lost and impaired sensibility the sensor/sensors can be incooperated in a glove which can be applied to the damaged hand. In order to make possible identification of separate fingers it is of course important that the sensors are applied to multiple fingers.

The vibrotactile stimuli which is registrated by the sensors when the hand or the prosthesis touches or slides over a surface is processed in an amplifying system which can be of stereophonic type. The signals are transmitted, by wires or via wireless techniques, to earphones applied to one or both ears. The hearing phones can be of small dimensions. The signal can also be transmitted directly to the bone of the cranium by stimulators placed, e.g. behind the external ear. Alternatively the signals can be transmitted directly to the bone of the cranium by implants introduced into the bone.

## Claims

1. A device for artificial sensibility where absent, lost or impaired sensibility in a hand prosthesis, hand, finger or other body-part is replaced by hearing perception of such a type that the surface, texture and in some cases also density and shape of a touched object can be perceived and identified, comprising:
- a hand prosthesis or a glove to be applied on said hand prosthesis, hand, finger or other body-part wherein a plurality of microphones are incorporated in said prosthesis or within said glove;
- an amplifying device;
- one or more earphones or other types of equipment for stimulation of the hearing sense;
wherein a vibrotactile stimulus, which is released by friction in association with touching an item in combination with movement of said hand prosthesis, hand, finger or other body-parts over said item, is registered by said microphones and is transmitted by said amplifying device to said one or more earphones or other types of equipment for stimulation of the hearing sense applied to one or both ears, or in association to one or both ears, whereby said vibrotactile stimulus is perceived as an acoustic apprehension.

2. A device for artificial sensibility as claimed in claim 1, wherein the acoustic signal is applied directly to the bone of the cranium by an external stimulation device or by an intraosseus implant.

3. A device for artificial sensibility as claimed in claims 1 or 2, wherein said microphones are applied in various positions in said hand prosthesis or said glove and said vibrotactile stimulus from the various positioned microphones is transmitted by a stereo amplifier and is projected to individually separated spatial positions so that the various locations on which sensors are applied can be individually identified by the acoustic signals and separated from each other.

4. A device for artificial sensibility as claimed in claims 1 to 3, whereby said device is combined with equipment for detection also of touch, temperature, movement or position of said hand prosthesis, hand, finger or other body-part.

5. A device for artificial sensibility as claimed in claims 1 to 4, wherein the transmission of said vibrotactile stimulus occurs by wireless transmission to one or both ears, or the bone of the cranium.

## Patentansprüche

1. Vorrichtung für künstliche Empfindlichkeit, bei der fehlende, verlorene oder beeinträchtigte Empfindlichkeit in einer Handprothese, einer Hand, einem Finger oder einem anderem Körperteil durch Höhrwahrnehmung solcher An ersetzt ist, dass die Oberfläche, Struktur und in einigen Fällen auch die Dichte und Form eines berührten Objekts wahrgenommen und identifiziert werden können, mit:
- einer Handprothese oder einem Handschuh angelegt an die Handprothese, die Hand, den Finger oder den anderen Körperteil, wobei eine Vielzahl von Mikrofonen in der Prothese oder dem Handschuh enthalten sind,
- einer Verstärkungsvorrichtung,
- einem oder mehreren Kopfhörern oder Einrichtungen anderer Art zur Stimulation des Gehörsinns,
wobei ein vibrotaktiler Reiz, der durch Reibung in Verbindung mit einer Berührung eines Gegenstands in Kombination mit einer Bewegung der Handprothese, der Hand, des Fingers oder des anderen Körperteils über den Gegenstand ausgelöst wird, durch die Mikrofone registriert und durch die Verstärkungsvorrichtung zu dem einen oder mehreren Kopfhörern oder der Einrichtung anderer Art zur Stimulation des Gehörsinns übertragen wird, die an ein oder beide Ohren angelegt oder in Verbindung mit einem oder beiden Ohren ist/sind, wodurch der vibrotaktile Reiz als eine akustische Wahrnehmung wahrgenommen wird.

2. Vorrichtung für künstliche Empfindlichkeit nach Anspruch 1, wobei, das akustische Signal durch eine externe Stimulationsvorrichtung oder durch ein intraosaäres Implantat direkt an den Knochen des Schädels angelegt wird.

3. Vorrichtung für künstliche Empfindlichkeit nach Anspruch 1 oder 2, wobei die Mikrofone in verschiedenen Positionen in der Handprothese oder dem Handschuh angebracht sind und der vibrotaktile Reiz von den verschieden positionierten Mikrofonen durch einen Stereoverstärker übertragen und auf individuelle getrennte räumliche Positionen projiziert wird, sodass die verschiedenen Orte, an denen die Sensoren angelegt sind, durch die akustischen Signale einzeln identifiziert und voneinander getrennt werden können.

4. Vorrichtung für künstliche Empfindlichkeit nach einem der Ansprüche 1 bis 3, wobei die Vorrichtung mit einer Einrichtung zum Feststellen auch von Berührung, Temperatur, Bewegung oder Position der Handprothese, der Hand, des Fingers oder des anderen Körperteils kombiniert ist.

5. Vorrichtung für künstliche Empfindlichkeit nach einem der Ansprüche 1 bis 4, wobei die Übertragung des vibrotaktilen Reizes durch drahtlose Übertragung zu einen oder beiden Ohren oder dem Knochen des Schädels erfolgt.

## Revendications

1. Dispositif de sensibilité artificielle selon lequel la sensibilité absente, perdue ou altérée dans une main prothétique, une main, un doigt ou une autre partie du corps est remplacée par la perception auditive d'un type tel que la surface, la texture et dans certains cas également la densité et la forme d'un objet touché peuvent être perçus et identifiés, comprenant :
une main prothétique ou un gant à appliquer sur ladite main prothétique, ladite main, ledit doigt ou ladite autre partie du corps dans laquelle un ensemble de microphones est incorporé dans ladite prothèse ou dans ledit gant ;
un appareil amplificateur ;
un ou plusieurs écouteurs ou autres types de matériel de stimulation du sens de l'audition ;
dans lequel un stimulus vibrotactile, qui est délivré par frottement associé au toucher d'un objet en combinaison avec le mouvement de ladite main prothétique, de ladite main, dudit doigt ou de ladite autre partie du corps sur ledit objet, est enregistré par lesdits microphones et est transmis par ledit appareil amplificateur au dit ou aux dits écouteurs ou autres types de matériel de stimulation du sens de l'audition appliqués à une ou aux deux oreilles, ou en association avec une ou deux oreilles, moyennant quoi ledit stimulus vibrotactile est perçu comme une appréhension acoustique.

2. Dispositif de sensibilité artificielle selon la revendication 1, dans lequel le signal acoustique est appliqué directement à l'os du crâne par un dispositif de stimulation externe ou par un implant intra-osseux.

3. Dispositif de sensibilité artificielle selon la revendication 1 ou 2, dans lequel lesdits microphones sont disposés en divers endroits dans ladite main prothétique ou dans ledit gant et ledit stimulus vibrotactile en provenance des divers microphones en place est transmis par un amplificateur stéréo et est projeté vers des positions spatiales séparées individuellement de telle sorte que les divers endroits sur lesquels les capteurs sont appliqués peuvent être identifiés individuellement par les signaux acoustiques et séparés les uns des autres.

4. Dispositif de sensibilité artificielle selon les revendications 1 à 3, moyennant quoi ledit dispositif est combiné avec un matériel de détection également du toucher, de la température, du mouvement ou de la position de ladite main prothétique, de ladite main, dudit doigt ou de ladite autre partie du corps.

5. Dispositif de sensibilité artificielle selon les revendications 1 à 4,dans lequel la transmission dudit stimulus vibrotactile se produit par transmission sans fil à une ou aux deux oreilles, ou à l'os du crâne.
